# EUROPEAN PATENT APPLICATION

(11) **EP 4 197 365 A1**
(43) Date of publication of application: **21.06.2023**
(21) Application number: 21215307.6
(22) Date of filing: 16.12.2021
(51) Int. Cl.: A24F 40/44

(54) **VAPOUR GENERATING SYSTEM**

(71) Applicant: IMPERIAL TOBACCO LIMITED, Bristol BS3 2LL (GB)
(72) Inventor: The designation of the inventor has not yet been filed
(74) Representative: Mewburn Ellis LLP

(57) **Abstract**

Provided is vapour generating system having a wick (162) comprising a first wicking material (162a) in contact with a second wicking material (162b). The first wicking material (162a) has a greater porosity than the second wicking material (162b), such that liquid is transported preferentially through the first wicking material (162a).

## Description

### Field of the Invention

The present invention relates to a vapour generating system for a smoking substitute apparatus and, in addition, a smoking substitute apparatus that is able to deliver nicotine to a user in an effective manner.

### Background

The smoking of tobacco is generally considered to expose a smoker to potentially harmful substances. It is thought that a significant amount of the potentially harmful substances are generated through the burning and/or combustion of the tobacco and the constituents of the burnt tobacco in the tobacco smoke itself.

Low temperature combustion of organic material such as tobacco is known to produce tar and other potentially harmful by-products. There have been proposed various smoking substitute systems in which the conventional smoking of tobacco is avoided.

Such smoking substitute systems can form part of nicotine replacement therapies aimed at people who wish to stop smoking and overcome a dependence on nicotine.

Known smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the health risks associated with conventional smoking.

In general, smoking substitute systems are intended to provide a substitute for the rituals of smoking, whilst providing the user with a similar, or improved, experience and satisfaction to those experienced with conventional smoking and with combustible tobacco products.

The use of smoking substitute systems has grown rapidly in the past few years as an aid to assist habitual smokers wishing to quit tobacco smoking. There are a number of different categories of smoking substitute systems, each utilising a different smoking substitute approach. Some smoking substitute systems are designed to resemble a conventional cigarette and are cylindrical in form with a mouthpiece at one end. Other smoking substitute devices do not generally resemble a cigarette (for example, the smoking substitute device may have a generally box-like form, in whole or in part).

One approach is the so-called "vaping" approach, in which a vaporisable liquid, or an aerosol former, sometimes typically referred to herein as "e-liquid", is heated by a heating device (sometimes referred to herein as an electronic cigarette or "e-cigarette" device) to produce an aerosol vapour which is inhaled by a user. The e-liquid typically includes a base liquid, nicotine and may include a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid and a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically couplable to a consumable including the tank and the heating element. In this way, when the tank of a consumable has been emptied of e-liquid, that consumable is removed from the main body and disposed of. The main body can then be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu^{™} e-cigarette. The myblu^{™} e-cigarette is a closed system which includes a main body and a consumable. The main body and consumable are physically and electrically coupled together by pushing the consumable into the main body. The main body includes a rechargeable battery. The consumable includes a mouthpiece and a sealed tank which contains e-liquid. The consumable further includes a heater, which for this device is a heating filament coiled around a portion of a wick. The wick is partially immersed in the e-liquid, and conveys e-liquid from the tank to the heating filament. The system is controlled by a microprocessor on board the main body. The system includes a sensor for detecting when a user is inhaling through the mouthpiece, the microprocessor then activating the device in response. When the system is activated, electrical energy is supplied from the power source to the heating device, which heats e-liquid from the tank to produce a vapour which is inhaled by a user through the mouthpiece.

### Summary of the Invention

It is apparent that the tank or reservoir which contains the e-liquid can only hold a certain amount of that liquid; once the liquid is used up the tank must be refilled or discarded. Accordingly maximum efficiency in utilizing all the e-liquid contained in the tank is a priority for sustainability and user satisfaction.

During usage, liquid in the tank is carried by the wick or other vaporiser element; it is inevitable that in certain conditions some of that liquid will be lost for example by leakage, displacement through sudden force application and so on. Obviously, that liquid cannot then be vapourised and is potentially wasted.

It is therefore desirable to limit such leakage and liquid loss.

The present disclosure has been devised in the light of the above considerations.

In a general aspect, the present inventors have found that a wick having two parts with different porosities can mean that liquid is carried preferentially in the part with higher porosity; accordingly, the parts can be arranged such that leakage from the higher porosity part is 'blocked' by the lower porosity part.

According to a first preferred aspect there is provided a vapour generating system for use in an electronic cigarette device, the system comprising: a reservoir, configured to retain a vapourisable liquid; a heating element; a wick, comprising a first wicking material in contact with a second wicking material, wherein the first wicking material comprises a first region in fluid contact with the reservoir and a second region in contact or near-contact with the heating element, and wherein the wick is configured to transport vapourisable liquid from the first region of the first wicking material to the second region via capillary action; characterised in that the first wicking material has a greater porosity than the second wicking material, such that liquid is transported preferentially through the first wicking material.

The wick may in some embodiments have a length along which the first wicking material and the second wicking material extend coaxially.

In some preferred embodiments, the first and second wicking materials are ceramic. This can permit closer control and design of the porosities of the first and second wicking materials.

In some embodiments the first and second wicking materials are separate materials. In other embodiments, the first and second wicking materials are integrally formed.

In such embodiments, the porosity of the wick may gradually change between the first wicking material and the second wicking material. Alternatively there may be a step change. For example, in some embodiments the porosity of the wick has at least two distinct portions, one of which corresponds to the first wicking material and one of which corresponds to the second wicking material, wherein each portion has a substantially uniform porosity.

It may be preferred that the porosity of the second wicking material is less than about 10%.

It may also be preferred that the porosity of the first wicking material is more than about 50%.

It may be preferred that the average pore diameter of the second wicking material is less than the average pore diameter of the first wicking material.

It may also be preferred that the average pore diameter of the second wicking material is less than about 50 nm.

It may also be preferred that the average pore diameter of the first wicking material is above about 75 nm.

A second preferred aspect of the invention provides a smoking substitute apparatus having: an air inlet; an outlet; a flow passage formed between the air inlet and the outlet; a vapour generating system as described herein; and a vaporisation chamber in communication with the flow passage, wherein at least part of the wick and the heater of the vapour generating system are positioned within the vaporization chamber.

The smoking substitute apparatus may be comprised by or within a cartridge configured for engagement with a main body, the cartridge and main body together forming a smoking substitute system. The smoking substitute apparatus may be removably engageable with the main body (which may also be referred to herein as the base unit).

The smoking substitute apparatus may be in the form of a consumable. The consumable may be configured for engagement with a main body. When the consumable is engaged with the main body, the combination of the consumable and the main body may form a smoking substitute system such as a closed smoking substitute system. For example, the consumable may comprise components of the system that are disposable, and the main body may comprise non-disposable or non-consumable components (e.g. power supply, controller, sensor, etc.) that facilitate the generation and/or delivery of aerosol by the consumable. In such an embodiment, the aerosol precursor (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol former (e.g. e-liquid) of the system may be replenished by re-filling, e.g. a reservoir of the smoking substitute apparatus, with the aerosol precursor (rather than replacing a consumable component of the apparatus).

In light of this, it should be appreciated that some of the features described herein as being part of the smoking substitute apparatus may alternatively form part of a main body for engagement with the smoking substitute apparatus. This may be the case in particular when the smoking substitute apparatus is in the form of a consumable.

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting, or the like.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

In the present invention, the vapour generating system comprises a reservoir configured to store an aerosol precursor, such as an e-liquid. The e-liquid may, for example, comprise a base liquid. The e-liquid may further comprise nicotine. The base liquid may include propylene glycol and/or vegetable glycerine. The e-liquid may be substantially flavourless. That is, the e-liquid may not contain any deliberately added additional flavourant and may consist solely of a base liquid of propylene glycol and/or vegetable glycerine and nicotine.

The reservoir may be in the form of a tank. At least a portion of the tank may be light-transmissive. For example, the tank may comprise a window to allow a user to visually assess the quantity of e-liquid in the tank. A housing of the smoking substitute apparatus may comprise a corresponding aperture (or slot) or window that may be aligned with a light-transmissive portion (e.g. window) of the tank. The reservoir may be referred to as a "clearomizer" if it includes a window, or a "cartomizer" if it does not.

The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage, for at least a part of the length of the passage. In this respect, the tank may surround the passage, e.g. in an annular arrangement around the passage.

The vaporisation chamber may be arranged to be in fluid communication with the inlet and outlet of the passage. The vaporisation chamber may be an enlarged portion of the passage. In this respect, the air as drawn in by the user may entrain the generated vapour in a flow away from heater. The entrained vapour may form an aerosol in the vaporisation chamber, or it may form the aerosol further downstream along the passage. The vaporisation chamber may be at least partially defined by the tank. The tank may substantially (or fully) define the vaporisation chamber, and thus may form the enclosure. In this respect, the tank may surround the vaporisation chamber, e.g. in an annular arrangement around the vaporisation chamber.

In use, the user may puff on a mouthpiece of the smoking substitute apparatus, i.e. draw on the smoking substitute apparatus by inhaling, to draw in an air stream therethrough. The part of the air flow which bypasses the vaporisation chamber (dilution air flow) may combine with the other part of the air flow (main air flow) for diluting the aerosol contained therein. The dilution air flow may be directly inhaled by the user without passing through the passage of the smoking substitute apparatus.

As a user puffs on the mouthpiece, vaporised e-liquid entrained in the passing air flow may be drawn towards the outlet of the passage. The vapour may cool, and thereby nucleate and/or condense along the passage to form a plurality of aerosol droplets, e.g. nicotine-containing aerosol droplets. A portion of these aerosol droplets may be delivered to and be absorbed at a target delivery site, e.g. a user's lung, whilst a portion of the aerosol droplets may instead adhere onto other parts of the user's respiratory tract, e.g. the user's oral cavity and/or throat. Typically, in some known smoking substitute apparatuses, the aerosol droplets as measured at the outlet of the passage, e.g. at the mouthpiece, may have a droplet size, dso, of less than 1µm.

In some embodiments of the invention, the dso particle size of the aerosol particles is preferably at least 1 µm, more preferably at least 2 µm. Typically, the dso particle size is not more than 10 µm, preferably not more than 9 µm, not more than 8 µm, not more than 7 µm, not more than 6 µm, not more than 5 µm, not more than 4 µm or not more than 3 µm. It is considered that providing aerosol particle sizes in such ranges permits improved interaction between the aerosol particles and the user's lungs.

The particle droplet size, dso, of an aerosol may be measured by a laser diffraction technique. For example, the stream of aerosol output from the outlet of the passage may be drawn through a Malvern Spraytec laser diffraction system, where the intensity and pattern of scattered laser light are analysed to calculate the size and size distribution of aerosol droplets. As will be readily understood, the particle size distribution may be expressed in terms of d₁₀, d₅₀ and d₉₀, for example. Considering a cumulative plot of the volume of the particles measured by the laser diffraction technique, the d₁₀ particle size is the particle size below which 10% by volume of the sample lies. The dso particle size is the particle size below which 50% by volume of the sample lies. The d₉₀ particle size is the particle size below which 90% by volume of the sample lies. Unless otherwise indicated herein, the particle size measurements are volume-based particle size measurements, rather than number-based or mass-based particle size measurements.

The spread of particle size may be expressed in terms of the span, which is defined as (d₉₀-d₁₀)/d₅₀. Typically, the span is not more than 20, preferably not more than 10, preferably not more than 8, preferably not more than 4, preferably not more than 2, preferably not more than 1, or not more than 0.5.

The smoking substitute apparatus (or main body engaged with the smoking substitute apparatus) may comprise a power source. The power source may be electrically connected (or connectable) to a heater of the smoking substitute apparatus (e.g. when the smoking substitute apparatus is engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface of the main body may be configured to transfer electrical power from the power source to a heater of the consumable via the electrical interface of the consumable.

The electrical interface of the smoking substitute apparatus may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an identification means, which may, for example, be in the form of an RFID reader, a barcode or QR code reader. This identification means may be able to identify a characteristic (e.g. a type) of a consumable engaged with the main body. In this respect, the consumable may include any one or more of an RFID chip, a barcode or QR code, or memory within which is an identifier and which can be interrogated via the identification means.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth^{®}. To this end, the wireless interface could include a Bluetooth^{®} antenna. Other wireless communication interfaces, e.g. WiFi^{®}, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may be provided in the consumable or alternatively may be provided in the main body.

The term "flavourant" is used to describe a compound or combination of compounds that provide flavour and/or aroma. For example, the flavourant may be configured to interact with a sensory receptor of a user (such as an olfactory or taste receptor). The flavourant may include one or more volatile substances.

The flavourant may be provided in solid or liquid form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The present inventors consider that a flow rate of 1.3 L min⁻¹ is towards the lower end of a typical user expectation of flow rate through a conventional cigarette and therefore through a user-acceptable smoking substitute apparatus. The present inventors further consider that a flow rate of 2.0 L min⁻¹ is towards the higher end of a typical user expectation of flow rate through a conventional cigarette and therefore through a user-acceptable smoking substitute apparatus. Embodiments of the present invention therefore provide an aerosol with advantageous particle size characteristics across a range of flow rates of air through the apparatus.

The aerosol may have a Dv50 of at least 1.1 µm, at least 1.2 µm, at least 1.3 µm, at least 1.4 µm, at least 1.5 µm, at least 1.6 µm, at least 1.7 µm, at least 1.8 µm, at least 1.9 µm or at least 2.0 µm.

The aerosol may have a Dv50 of not more than 4.9 µm, not more than 4.8 µm, not more than 4.7 µm, not more than 4.6 µm, not more than 4.5 µm, not more than 4.4 µm, not more than 4.3 µm, not more than 4.2 µm, not more than 4.1 µm, not more than 4.0 µm, not more than 3.9 µm, not more than 3.8 µm, not more than 3.7 µm, not more than 3.6 µm, not more than 3.5 µm, not more than 3.4 µm, not more than 3.3 µm, not more than 3.2 µm, not more than 3.1 µm or not more than 3.0 µm.

A particularly preferred range for Dv50 of the aerosol is in the range 2-3 µm.

The air inlet, flow passage, outlet, and the vaporisation chamber may be configured so that, when the air flow rate inhaled by the user through the apparatus is 1.3 L min⁻¹, the average magnitude of velocity of air in the vaporisation chamber is in the range 0-1.3 ms⁻¹. The average magnitude velocity of air may be calculated based on knowledge of the geometry of the vaporisation chamber, and the flow rate.

When the air flow rate inhaled by the user through the apparatus is 1.3 L min⁻¹, the average magnitude of velocity of air in the vaporisation chamber may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min⁻¹, the average magnitude of velocity of air in the vaporisation chamber may be at most 1.2 ms⁻¹, at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

The air inlet, flow passage, outlet, and the vaporisation chamber may be configured so that, when the air flow rate inhaled by the user through the apparatus is 2.0 L min⁻¹, the average magnitude of velocity of air in the vaporisation chamber is in the range 0-1.3 ms⁻¹. The average magnitude velocity of air may be calculated based on knowledge of the geometry of the vaporisation chamber, and the flow rate.

When the airflow rate inhaled by the user through the apparatus is 2.0 L min⁻¹, the average magnitude of velocity of air in the vaporisation chamber may be at least 0.001 ms⁻¹, or at least 0.005 ms⁻¹, or at least 0.01 ms⁻¹, or at least 0.05 ms⁻¹.

When the airflow rate inhaled by the user through the apparatus is 2.0 L min⁻¹, the average magnitude of velocity of air in the vaporisation chamber may be at most 1.2 ms⁻¹, at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

When the calculated average magnitude of velocity of air in the vaporisation chamber is in the ranges specified, it is considered that the resultant aerosol particle size is advantageously controlled to be in a desirable range. It is further considered that the configuration of the apparatus can be selected so that the average magnitude of velocity of air in the vaporisation chamber can be brought within the ranges specified, at the exemplary flow rate of 1.3 L min⁻¹ and/or the exemplary flow rate of 2.0 L min⁻¹.

The aerosol generator described herein comprises a vaporiser element which can be loaded with aerosol precursor, the vaporiser element being heatable by a heater and presenting a vaporiser element surface to air in the vaporisation chamber. A vaporiser element region may be defined as a volume extending outwardly from the vaporiser element surface to a distance of 1 mm from the vaporiser element surface.

The air inlet, flow passage, outlet, and the vaporisation chamber may be configured so that, when the air flow rate inhaled by the user through the apparatus is 1.3 L min⁻¹, the average magnitude of velocity of air in the vaporiser element region is in the range 0-1.2 ms⁻¹. The average magnitude of velocity of air in the vaporiser element region may be calculated using computational fluid dynamics.

When the air flow rate inhaled by the user through the apparatus is 1.3 L min⁻¹, the average magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min⁻¹, the average magnitude of velocity of air in the vaporiser element region may be at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

The air inlet, flow passage, outlet, and the vaporisation chamber may be configured so that, when the air flow rate inhaled by the user through the apparatus is 2.0 L min⁻¹, the average magnitude of velocity of air in the vaporiser element region is in the range 0-1.2 ms⁻¹. The average magnitude of velocity of air in the vaporiser element region may be calculated using computational fluid dynamics.

When the air flow rate inhaled by the user through the apparatus is 2.0 L min⁻¹, the average magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 2.0 L min⁻¹, the average magnitude of velocity of air in the vaporiser element region may be at most 1.1 ms⁻¹, at most 1.0 ms⁻¹, at most 0.9 ms⁻¹, at most 0.8 ms⁻¹, at most 0.7 ms⁻¹ or at most 0.6 ms⁻¹.

When the average magnitude of velocity of air in the vaporiser element region is in the ranges specified, it is considered that the resultant aerosol particle size is advantageously controlled to be in a desirable range. It is further considered that the velocity of air in the vaporiser element region is more relevant to the resultant particle size characteristics than consideration of the velocity in the vaporisation chamber as a whole. This is in view of the significant effect of the velocity of air in the vaporiser element region on the cooling of the vapour emitted from the vaporiser element surface.

Additionally or alternatively is it relevant to consider the maximum magnitude of velocity of air in the vaporiser element region.

Therefore, the air inlet, flow passage, outlet, and the vaporisation chamber may be configured so that, when the air flow rate inhaled by the user through the apparatus is 1.3 L min⁻¹, the maximum magnitude of velocity of air in the vaporiser element region is in the range 0-2.0 ms⁻¹.

When the air flow rate inhaled by the user through the apparatus is 1.3 L min⁻¹, the maximum magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 1.3 L min⁻¹, the maximum magnitude of velocity of air in the vaporiser element region may be at most 1.9 ms⁻¹, at most 1.8 ms⁻¹, at most 1.7 ms⁻¹, at most 1.6 ms⁻¹, at most 1.5 ms⁻¹, at most 1.4 ms⁻¹, at most 1.3 ms⁻¹ or at most 1.2 ms⁻¹.

The air inlet, flow passage, outlet, and the vaporisation chamber may be configured so that, when the air flow rate inhaled by the user through the apparatus is 2.0 L min⁻¹, the maximum magnitude of velocity of air in the vaporiser element region is in the range 0-2.0 ms⁻¹.

When the air flow rate inhaled by the user through the apparatus is 2.0 L min⁻¹, the maximum magnitude of velocity of air in the vaporiser element region may be at least 0.001 ms⁻¹, or at least 0.005 ms⁻¹, or at least 0.01 ms⁻¹, or at least 0.05 ms⁻¹.

When the air flow rate inhaled by the user through the apparatus is 2.0 L min⁻¹, the maximum magnitude of velocity of air in the vaporiser element region may be at most 1.9 ms⁻¹, at most 1.8 ms⁻¹, at most 1.7 ms⁻¹, at most 1.6 ms⁻¹, at most 1.5 ms⁻¹, at most 1.4 ms⁻¹, at most 1.3 ms⁻¹ or at most 1.2 ms⁻¹.

It is considered that configuring the apparatus in a manner to permit such control of velocity of the airflow at the vaporiser permits the generation of aerosols with particularly advantageous particle size characteristics, including Dv50 values.

Additionally or alternatively is it relevant to consider the turbulence intensity in the vaporiser chamber in view of the effect of turbulence on the particle size of the generated aerosol. For example, the air inlet, flow passage, outlet, and the vaporisation chamber may be configured so that, when the air flow rate inhaled by the user through the apparatus is 1.3 L min⁻¹, the turbulence intensity in the vaporiser element region is not more than 1%.

When the air flow rate inhaled by the user through the apparatus is 1.3 L min⁻¹, the turbulence intensity in the vaporiser element region may be not more than 0.95%, not more than 0.9%, not more than 0.85%, not more than 0.8%, not more than 0.75%, not more than 0.7%, not more than 0.65% or not more than 0.6%.

It is considered that configuring the apparatus in a manner to permit such control of the turbulence intensity in the vaporiser element region permits the generation of aerosols with particularly advantageous particle size characteristics, including Dv50 values.

Following detailed investigations, the inventors consider, without wishing to be bound by theory, that the particle size characteristics of the generated aerosol may be determined by the cooling rate experienced by the vapour after emission from the vaporiser element (e.g. wick). In particular, it appears that imposing a relatively slow cooling rate on the vapour has the effect of generating aerosols with a relatively large particle size. The parameters discussed above (velocity and turbulence intensity) are considered to be mechanisms for implementing a particular cooling dynamic to the vapour.

More generally, it is considered that the air inlet, flow passage, outlet, and the vaporisation chamber may be configured so that a desired cooling rate is imposed on the vapour. The particular cooling rate to be used depends of course on the nature of the aerosol precursor and other conditions. However, for a particular aerosol precursor it is possible to define a set of testing conditions in order to define the cooling rate, and by extension this imposes limitations on the configuration of the apparatus to permit such cooling rates as are shown to result in advantageous aerosols. Accordingly, the air inlet, flow passage, outlet, and the vaporisation chamber may be configured so that the cooling rate of the vapour is such that the time taken to cool to 50 °C is not less than 16 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 1.3 L min⁻¹.

Additionally or alternatively, the air inlet, flow passage, outlet, and the vaporisation chamber may be configured so that the cooling rate of the vapour is such that the time taken to cool to 50 °C is not less than 16 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 2.0 L min⁻¹.

Cooling of the vapour such that the time taken to cool to 50 °C is not less than 16 ms corresponds to an equivalent linear cooling rate of not more than 10 °C/ms.

The equivalent linear cooling rate of the vapour to 50 °C may be not more than 9 °C/ms, not more than 8 °C/ms, not more than 7 °C/ms, not more than 6 °C/ms or not more than 5 °C/ms.

Cooling of the vapour such that the time taken to cool to 50 °C is not less than 32 ms corresponds to an equivalent linear cooling rate of not more than 5 °C/ms.

The testing protocol set out above considers the cooling of the vapour (and subsequent aerosol) to a temperature of 50 °C. This is a temperature which can be considered to be suitable for an aerosol to exit the apparatus for inhalation by a user without causing significant discomfort. It is also possible to consider cooling of the vapour (and subsequent aerosol) to a temperature of 75 °C. Although this temperature is possibly too high for comfortable inhalation, it is considered that the particle size characteristics of the aerosol are substantially settled by the time the aerosol cools to this temperature (and they may be settled at still higher temperature).

Accordingly, the air inlet, flow passage, outlet, and the vaporisation chamber may be configured so that the cooling rate of the vapour is such that the time taken to cool to 75 °C is not less than 4.5 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 1.3 L min⁻¹.

Additionally or alternatively, the air inlet, flow passage, outlet, and the vaporisation chamber may be configured so that the cooling rate of the vapour is such that the time taken to cool to 75 °C is not less than 4.5 ms, when tested according to the following protocol. The aerosol precursor is an e-liquid consisting of 1.6% freebase nicotine and the remainder a 65:35 propylene glycol and vegetable glycerine mixture, the e-liquid having a boiling point of 209 °C. Air is drawn into the air inlet at a temperature of 25 °C. The vaporiser is operated to release a vapour of total particulate mass 5 mg over a 3 second duration from the vaporiser element surface in an air flow rate between the air inlet and outlet of 2.0 L min⁻¹.

Cooling of the vapour such that the time taken to cool to 75 °C is not less than 4.5 ms corresponds to an equivalent linear cooling rate of not more than 30 °C/ms.

The equivalent linear cooling rate of the vapour to 75 °C may be not more than 29 °C/ms, not more than 28 °C/ms, not more than 27 °C/ms, not more than 26 °C/ms, not more than 25 °C/ms, not more than 24 °C/ms, not more than 23 °C/ms, not more than 22 °C/ms, not more than 21 °C/ms, not more than 20 °C/ms, not more than 19 °C/ms, not more than 18 °C/ms, not more than 17 °C/ms, not more than 16 °C/ms, not more than 15 °C/ms, not more than 14 °C/ms, not more than 13 °C/ms, not more than 12 °C/ms, not more than 11 °C/ms or not more than 10 °C/ms.

Cooling of the vapour such that the time taken to cool to 75 °C is not less than 13 ms corresponds to an equivalent linear cooling rate of not more than 10 °C/ms.

It is considered that configuring the apparatus in a manner to permit such control of the cooling rate of the vapour permits the generation of aerosols with particularly advantageous particle size characteristics, including Dv50 values.

The invention includes the combination of the aspects and preferred features described except where such a combination is clearly impermissible or expressly avoided.

### Summary of the Figures

Figure 1 is a schematic front view of a smoking substitute system, according to a first reference arrangement, in an engaged position;
Figure 2 is a schematic front view of the smoking substitute system of the first reference arrangement in a disengaged position;
Figure 3 is a schematic longitudinal cross sectional view of a smoking substitute apparatus of the first reference arrangement;
Figure 4 is an enlarged schematic cross sectional view of part of the air passage and vaporisation chamber of the first reference arrangement;
Figure 5 shows a schematic cross sectional view of a smoking substitute apparatus of a second reference arrangement;
Figure 6 shows a schematic cross sectional view of a smoking substitute apparatus of a third reference arrangement.
Figure 7A, B and C show cross sectional views through different regions of wicks in several embodiments of the present invention.

### Detailed Description of the Invention

Further background to the present invention and further aspects and embodiments of the present invention will now be discussed with reference to the accompanying figures. Further aspects and embodiments will be apparent to those skilled in the art. The contents of all documents mentioned in this text are incorporated herein by reference in their entirety.

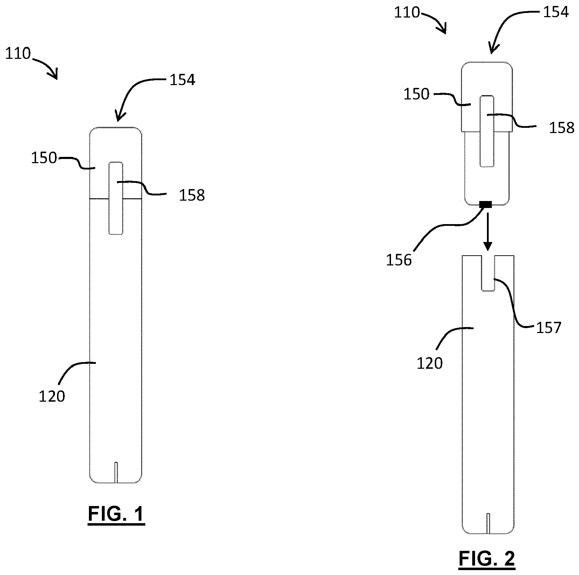

Figures 1 and 2 illustrate a smoking substitute system in the form of an e-cigarette system 110. The system 110 comprises a main body 120 of the system 110, and a smoking substitute apparatus in the form of an e-cigarette consumable (or "pod") 150. In the illustrated reference arrangement the consumable 150 (sometimes referred to herein as a smoking substitute apparatus) is removable from the main body 120, so as to be a replaceable component of the system 110. The e-cigarette system 110 is a closed system in the sense that it is not intended that the consumable should be refillable with e-liquid by a user.

As is apparent from Figures 1 and 2, the consumable 150 is configured to engage the main body 120. Figure 1 shows the main body 120 and the consumable 150 in an engaged state, whilst Figure 2 shows the main body 120 and the consumable 150 in a disengaged state. When engaged, a portion of the consumable 150 is received in a cavity of corresponding shape in the main body 120 and is retained in the engaged position by way of a snap-engagement mechanism. In other embodiments, the main body 120 and consumable 150 may be engaged by screwing one into (or onto) the other, or through a bayonet fitting, or by way of an interference fit.

The system 110 is configured to vaporise an aerosol precursor, which in the illustrated reference arrangement is in the form of a nicotine-based e-liquid 160. The e-liquid 160 comprises nicotine and a base liquid including propylene glycol and/or vegetable glycerine. In the present embodiment, the e-liquid 160 is flavoured by a flavourant. In other embodiments, the e-liquid 160 may be flavourless and thus may not include any added flavourant.

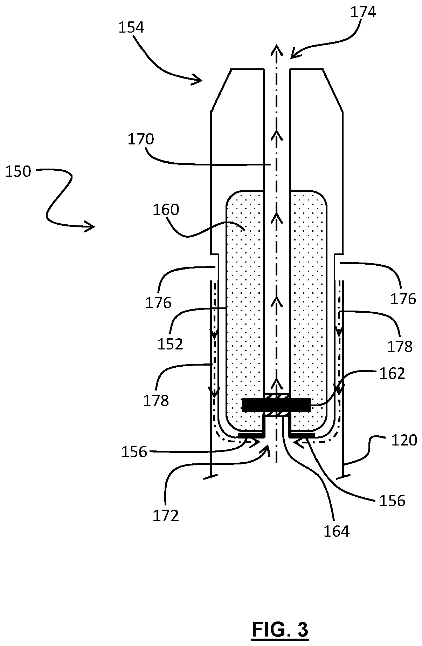

Figure 3 shows a schematic longitudinal cross sectional view of a reference arrangement of the smoking substitute apparatus forming part of the smoking substitute system shown in Figures 1 and 2. In Figure 3, the e-liquid 160 is stored within a reservoir in the form of a tank 152 that forms part of the consumable 150. In the illustrated reference arrangement, the consumable 150 is a "single-use" consumable 150. That is, upon exhausting the e-liquid 160 in the tank 152, the intention is that the user disposes of the entire consumable 150. The term "single-use" does not necessarily mean the consumable is designed to be disposed of after a single smoking session. Rather, it defines the consumable 150 is not arranged to be refilled after the e-liquid contained in the tank 152 is depleted. The tank may include a vent (not shown) to allow ingress of air to replace e-liquid that has been used from the tank. The consumable 150 preferably includes a window 158 (see Figures 1 and 2), so that the amount of e-liquid in the tank 152 can be visually assessed. The main body 120 includes a slot 157 so that the window 158 of the consumable 150 can be seen whilst the rest of the tank 152 is obscured from view when the consumable 150 is received in the cavity of the main body 120. The consumable 150 may be referred to as a "clearomizer" when it includes a window 158, or a "cartomizer" when it does not.

In some embodiments, the e-liquid (i.e. aerosol precursor) may be the only part of the system that is truly "single-use". That is, the tank may be refillable with e-liquid or the e-liquid may be stored in a non-consumable component of the system. For example, in such embodiments, the e-liquid may be stored in a tank located in the main body or stored in another component that is itself not single-use (e.g. a refillable cartomizer).

The external wall of tank 152 is provided by a casing of the consumable 150. The tank 152 annularly surrounds, and thus defines a portion of, a passage 170 that extends between a vaporiser inlet 172 and an outlet 174 at opposing ends of the consumable 150. In this respect, the passage 170 comprises an upstream end at the end of the consumable 150 that engages with the main body 120, and a downstream end at an opposing end of the consumable 150 that comprises a mouthpiece 154 of the system 110.

When the consumable 150 is received in the cavity of the main body 120 as shown in Figure 3, a plurality of device air inlets 176 are formed at the boundary between the casing of the consumable and the casing of the main body. The device air inlets 176 are in fluid communication with the vaporiser inlet 172 through an inlet flow channel 178 formed in the cavity of the main body which is of corresponding shape to receive a part of the consumable 150. Air from outside of the system 110 can therefore be drawn into the passage 170 through the device air inlets 176 and the inlet flow channels 178.

When the consumable 150 is engaged with the main body 120, a user can inhale (i.e. take a puff) via the mouthpiece 154 so as to draw air through the passage 170, and so as to form an airflow (indicated by the dashed arrows in Figure 3) in a direction from the vaporiser inlet 172 to the outlet 174. Although not illustrated, the passage 170 may be partially defined by a tube (e.g. a metal tube) extending through the consumable 150. In Figure 3, for simplicity, the passage 170 is shown with a substantially circular cross-sectional profile with a constant diameter along its length. In some embodiments, the passage may have other cross-sectional profiles, such as oval shaped or polygonal shaped profiles. Further, in other embodiments, the cross sectional profile and the diameter (or hydraulic diameter) of the passage may vary along its longitudinal axis.

The smoking substitute system 110 is configured to vaporise the e-liquid 160 for inhalation by a user. To provide this operability, the consumable 150 comprises a heater having a porous wick 162 and a resistive heating element in the form of a heating filament 164 that is helically wound (in the form of a coil) around a portion of the porous wick 162. The porous wick 162 extends across the passage 170 (i.e. transverse to a longitudinal axis of the passage 170 and thus also transverse to the airflow along the passage 170 during use) and opposing ends of the wick 162 extend into the tank 152 (so as to be immersed in the e-liquid 160). In this way, e-liquid 160 contained in the tank 152 is conveyed from the opposing ends of the porous wick 162 to a central portion of the porous wick 162 so as to be exposed to the airflow in the passage 170.

The helical filament 164 is wound about the exposed central portion of the porous wick 162 and is electrically connected to an electrical interface in the form of electrical contacts 156 mounted at the end of the consumable that is proximate the main body 120 (when the consumable and the main body are engaged). When the consumable 150 is engaged with the main body 120, electrical contacts 156 make contact with corresponding electrical contacts (not shown) of the main body 120. The main body electrical contacts are electrically connectable to a power source (not shown) of the main body 120, such that (in the engaged position) the filament 164 is electrically connectable to the power source. In this way, power can be supplied by the main body 120 to the filament 164 in order to heat the filament 164. This heats the porous wick 162 which causes e-liquid 160 conveyed by the porous wick 162 to vaporise and thus to be released from the porous wick 162. The vaporised e-liquid becomes entrained in the airflow and, as it cools in the airflow (between the heated wick and the outlet 174 of the passage 170), condenses to form an aerosol. This aerosol is then inhaled, via the mouthpiece 154, by a user of the system 110. As e-liquid is lost from the heated portion of the wick, further e-liquid is drawn along the wick from the tank to replace the e-liquid lost from the heated portion of the wick.

The filament 164 and the exposed central portion of the porous wick 162 are positioned across the passage 170. More specifically, the part of passage that contains the filament 164 and the exposed portion of the porous wick 162 forms a vaporisation chamber. In the illustrated example, the vaporisation chamber has the same cross-sectional diameter as the passage 170. However, in some embodiments the vaporisation chamber may have a different cross sectional profile compared with the passage 170. For example, the vaporisation chamber may have a larger cross sectional diameter than at least some of the downstream part of the passage 170 so as to enable a longer residence time for the air inside the vaporisation chamber.

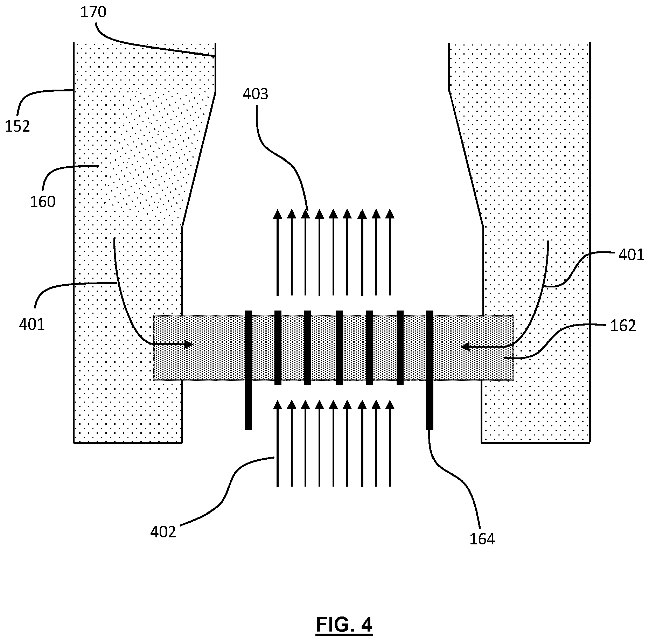

Figure 4 illustrates in more detail the vaporisation chamber and therefore the region of the consumable 150 around the wick 162 and filament 164. The helical filament 164 is wound around a central portion of the porous wick 162. The porous wick extends across passage 170. E-liquid 160 contained within the tank 152 is conveyed as illustrated schematically by arrows 401, i.e. from the tank and towards the central portion of the porous wick 162.

When the user inhales, air is drawn from through the inlets 176 shown in Figure 3, along inlet flow channel 178 to vaporisation chamber inlet 172 and into the vaporisation chamber containing porous wick 162. The porous wick 162 extends substantially transverse to the airflow direction. The airflow passes around the porous wick, at least a portion of the airflow substantially following the surface of the porous wick 162. In examples where the porous wick has a cylindrical cross-sectional profile, the airflow may follow a curved path around an outer periphery of the porous wick 162.

At substantially the same time as the airflow passes around the porous wick 162, the filament 164 is heated so as to vaporise the e-liquid which has been wicked into the porous wick. The airflow passing around the porous wick 162 picks up this vaporised e-liquid, and the vapour-containing airflow is drawn in direction 403 further down passage 170.

The power source of the main body 120 may be in the form of a battery (e.g. a rechargeable battery such as a lithium ion battery). The main body 120 may comprise a connector in the form of e.g. a USB port for recharging this battery. The main body 120 may also comprise a controller that controls the supply of power from the power source to the main body electrical contacts (and thus to the filament 164). That is, the controller may be configured to control a voltage applied across the main body electrical contacts, and thus the voltage applied across the filament 164. In this way, the filament 164 may only be heated under certain conditions (e.g. during a puff and/or only when the system is in an active state). In this respect, the main body 120 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 120 and consumable 150 may comprise a further interface which may, for example, be in the form of an RFID reader, a barcode or QR code reader. This interface may be able to identify a characteristic (e.g. a type) of a consumable 150 engaged with the main body 120. In this respect, the consumable 150 may include any one or more of an RFID chip, a barcode or QR code, or memory within which is an identifier and which can be interrogated via the interface.

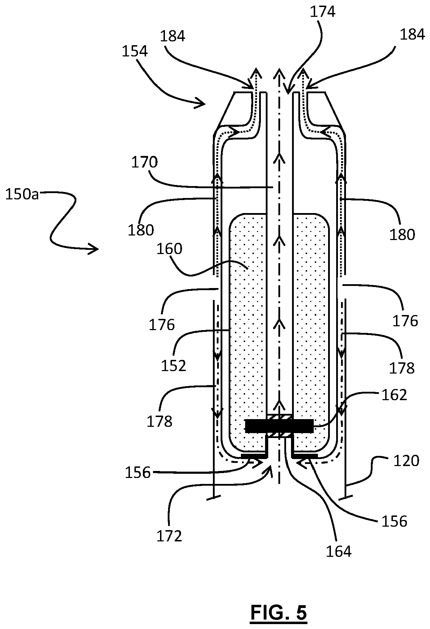

An apparatus according to an embodiment of the invention may be configured such that in use, at least part of the air flow drawn by a user through the apparatus from the air inlet to the outlet bypasses the vaporisation chamber defined by the enclosure. A second reference arrangement of an apparatus, shown in Figure 5, provides an example of how such a bypassing air flow may be created. Accordingly, some embodiments of the invention may include one or a combination of the features of the second reference arrangement (and variations thereof) where such features are combinable with the present invention. This second reference arrangement is described below.

Figure 5 illustrates a schematic longitudinal cross sectional view of a second reference arrangement of the smoking substitute apparatus forming part of the smoking substitute system shown in Figures 1 and 2. The arrangement illustrated in Figure 5 differs from the first reference arrangement illustrated in Figure 3 in that the substitute smoking apparatus includes two bypass passages 180 in addition to the vaporiser passage 170. The bypass air passages extend between the plurality of device air inlets 176 and two outlets 184. In other variations of the second reference arrangement, the number of bypass passages 180 and corresponding outlets 184 may be greater or smaller than in the illustrated example. Furthermore, there may be more or fewer air inlets and there may be more or fewer outlets.

In Figure 5 for simplicity, the bypass passage 180 is shown with a substantially circular cross-sectional profile with a constant diameter along its length. In some variations of the second reference arrangement, the bypass passage 180 may have other cross-sectional profiles, such as oval shaped or polygonal shaped profiles. Further, in some variations of the second reference arrangement, the cross sectional profile and the diameter (or hydraulic diameter) of the bypass passage 180 may vary along its longitudinal axis.

The provision of a bypass passage 180 means that a part of the air drawn through the smoking substitute apparatus 150a when a user inhales via the mouthpiece 154 is not drawn through the vaporisation chamber. This has the effect of reducing the flow rate through the vaporisation chamber in correspondence with the respective flow resistances presented by the vaporiser passage 170 and the bypass passage 180. This can reduce the correlation between the flow rate through the smoking substitute apparatus 150a (i.e. the user's draw rate) and the particle size generated when the e-liquid 160 is vaporised and subsequently forms an aerosol. Therefore, the smoking substitute apparatus 150a of the second reference arrangement can deliver a more consistent aerosol to a user.

Furthermore, the smoking substitute apparatus 150a of the second reference arrangement is capable of producing an increased particle droplet size, dso, based on typical inhalation rates undertaken by a user, compared to the first reference arrangement of Figure 3. Such larger droplet sizes may be beneficial for the delivery of vapour to a user's lungs. The preferred ratio between the dimensions of the bypass passage 180 and the dimensions of the vaporiser passage 170, and hence flow rate in the respective passages may be determined from representative user inhalation rates and from the required air flow rate through the vaporisation chamber to deliver a desired droplet size. For example, an average total flow rate of 1.3 litres per minute may be split such that 0.8 litres per minute passes through the bypass air channel 180, and 0.5 litres per minute passes through the vaporiser channel 170, a bypass:vaporiser flow rate ratio of 1.6:1. Such a flow rate may provide an average droplet size, dso, of 1-3 µm (more preferably 2-3 µm) with a span of not more than 20 (preferably not more than 10). Alternative flow rate ratios may be provided based on calculations and measurements of user flow rate, vaporiser flow rate, and average droplet size dso. A bypass:vaporiser flow rate ratio of between 0.5:1 and 20:1, typically at an average total flow rate of 1.3 litres per minute may be advantageous depending on the configuration of the smoking substitute apparatus.

The bypass passage and vaporiser passage extend from a common device inlet 176. This has the benefit of ensuring more consistent airflow through the bypass passage 180 and vaporiser passage 170 across the lifetime of the smoking substitute apparatus 150a, since any obstruction that impinges on an air inlet 176 will affect the airflow through both passages equally. The impact of inlet manufacturing variations can also be reduced for the same reason. This can therefore improve the user experience for the smoking substitute apparatus 150a. Furthermore, the provision of a common device inlet 176 simplifies the construction and external appearance of the device.

The bypass passage 180 and vaporiser passage 170 separate upstream of the vaporisation chamber. Therefore, no vapour is drawn through the bypass passage 180. Furthermore, because the bypass passage leads to outlet 184 that is separate from outlet 174 of the vaporiser passage, substantially no mixing of the bypass air and vaporiser air occurs within the smoking substitute apparatus 150a. Such mixing could otherwise lead to excessive cooling of the vapour and hence a build-up of condensation within the smoking substitute apparatus 150a. Such condensation could have adverse implications for delivering vapour to the user, for example by causing the user to draw liquid droplets rather than vapour when "puffing" on the mouthpiece 154.

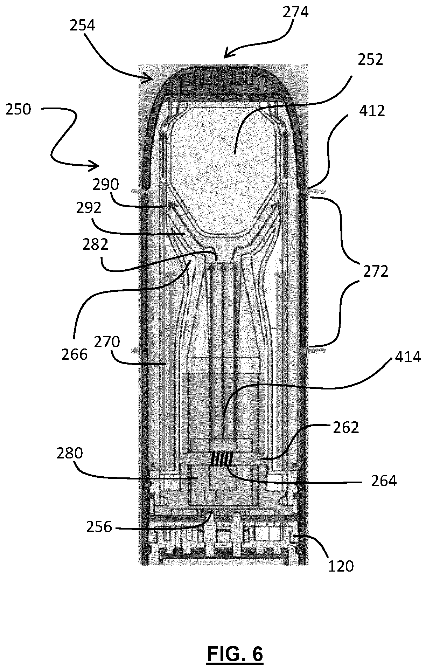

A further example of a bypass air flow is presented by a third reference arrangement. Accordingly, in some embodiments, the apparatus may include one or a combination of features of a third reference arrangement (and variations thereof), shown schematically in Figure 6, where such features are combinable with the present invention. This third reference arrangement is described below.

Figure 6 illustrates a longitudinal cross sectional view of a consumable 250 according to a further arrangement. In Figure 6, the consumable 250 is shown attached, at a first end of the consumable 250, to the main body 120 of Figure 1 and Figure 2. More specifically, the consumable 250 is configured to engage and disengage with the main body 120 and is interchangeable with the first reference arrangement 150 as shown in Figures 3 and 4. Furthermore, the consumable 250 is configured to interact with the main body 120 in the same manner as the first reference arrangement 150 and the user may operate the consumable 250 in the same manner as the first reference arrangement 150.

The consumable 250 comprises a housing. The consumable 250 comprises an aerosol generation chamber 280 in the housing. As shown in Figure 6, the aerosol generation chamber 280 takes the form of an open ended container, or a cup, with a single chamber outlet 282 opened towards the outlet 274 of the consumable 250.

In the illustrated third reference arrangement, the housing has a plurality of air inlets 272 defined or opened at the sidewall of the housing. An outlet 274 is defined or opened at a second end of the consumable 250 that comprises a mouthpiece 254. A pair of passages 270 each extend between the respective air inlets 272 and the outlet 274 to provide flow passage for an air flow 412 as a user puffs on the mouthpiece 254. The chamber outlet 282 is configured to be in fluid communication with the passages 270. The passages 270 extend from the air inlets 272 towards the first end of the consumable 250 before routing back to towards the outlet 274 at the second end of the consumable 250. That is, a portion of each of the passages 270 axially extends alongside the aerosol generation chamber 280. The path of the air flow path 412 is illustrated in Figure 6. In variations of the third reference arrangement, the passages 270 may extend from the air inlet 272 directly to the outlet 274 without routing towards the first end of consumable 250, e.g. the passages 270 may not axially extend alongside the aerosol generation chamber 280.

In some other variations of the third reference arrangement, the housing may not be provided with any air inlet for an air flow to enter the housing. For example, the chamber outlet may be directly connected to the outlet of the housing by an aerosol passage and therefore said aerosol passage may only convey aerosol as generated in the aerosol generation chamber. In these variations, the discharge of aerosol may be driven at least in part by the pressure increase during vaporisation of aerosol form.

Referring back to the third reference arrangement of Figure 6, the chamber outlet 282 is positioned downstream from the heater in the direction of the vapour and/or aerosol flow 414 and serves as the only gas flow passage to the internal volume of the aerosol generation chamber 280. In other words, the aerosol generation chamber 280 is sealed against air flow except for having the chamber outlet 282 in communication with the passages 270, the chamber outlet 282 permitting, in use, aerosol generated by the heater to be entrained into an air flow along the passage 270. In some other variations of the third reference arrangement, the sealed aerosol generation chamber 280 may comprise a plurality of chamber outlets 282 each arranged in fluid commutation with the passages 270. In the illustrated third reference arrangement, the aerosol generation chamber 280 does not comprise any aperture upstream of the heater that may serve as an air flow inlet (although in some arrangements a vent may be provided). In contrast with the consumable 150 as shown in Figures 3 and 4, the passages 270 of the consumable 250 allow the air flow, e.g. an entire amount of air flow, entering the housing to bypass the aerosol generation chamber 280. Such arrangement allows aerosol precursor to be vaporised in absence of the air flow. Therefore, the aerosol generation chamber may be considered to be a "stagnant" chamber. For example, the volumetric flowrate of vapour and/or aerosol in the aerosol generation chamber is configured to be less than 0.1 litre per minute. The vaporised aerosol precursor may cool and therefore condense to form an aerosol in the aerosol generation chamber 280, which is subsequently expulsed into or entrained with the air flow in passages 270. In addition, a portion of the vaporised aerosol precursor may remain as a vapour before leaving the aerosol generation chamber 280, and subsequently forms an aerosol as it is cooled by the air flow in the passages 270. The flow path of the vapour and/or aerosol 414 is illustrated in Figure 6.

In the illustrated third reference arrangement, the chamber outlet 282 is configured to be in fluid communication with a junction 290 at each of the passages 270 through a respective vapour channel 292. The junctions 290 merge the vapour channels 292 with their respective passages 270 such that vapour and/or aerosol formed in the aerosol generation chamber 280 may expand or entrain into the passages 270 through junction inlets of said junctions 290. The vapour channels form a buffering volume to minimise the amount of air flow that may back flow into the aerosol generation chamber 280. In some other variations of the third reference arrangement (not illustrated), the chamber outlet 282 may directly open towards the junction 290 at the passage, and therefore in such variations the vapour channel 292 may be omitted.

In some variations of the third reference arrangement (not illustrated), the chamber outlet may be closed by a one way valve. Said one way valve may be configured to allow a one way flow passage for the vapour and/or aerosol to be discharged from the aerosol generation chamber, and to reduce or prevent the air flow in the passages from entering the aerosol generation chamber.

In the illustrated third reference arrangement, the aerosol generation chamber 280 is configured to have a length of 20mm and a volume of 680mm³. The aerosol generation chamber is configured to allow vapour to be expulsed through the chamber outlet at a rate greater than 0.1 mg/second. In other variations of the third reference arrangement the aerosol generation chamber may be configured to have an internal volume ranging between 68mm³ to 680mm³, wherein the length of the aerosol generation chamber may range between 2mm to 20mm.

As shown in Figure 6, a part of each of the passages 270 axially extends alongside the aerosol generation chamber 280. For example, the passages 270 are formed between the aerosol generation chamber 280 and the housing. Such an arrangement reduces heat transfer from the aerosol generation chamber 280 to the external surfaces of the housing.

The aerosol generation chamber 280 comprises a heater extending across its width. The heater comprises a porous wick 262 and a heating filament 264 helically wound around a portion of the porous wick 162. A tank 252 is provided in the space between the aerosol generation chamber 280 and the outlet 274, the tank being for storing a reservoir of aerosol precursor. Therefore in contrast with the reference arrangement as shown in Figures 3 and 4, the tank 252 in the third reference arrangement does not substantially surround the aerosol generation chamber nor the passage 270. Instead, as shown in Figure 6, the tank is substantially positioned above the aerosol generation chamber 280 and the porous wick 262 when the consumable 250 is placed in an upright orientation during use. The end portions of the porous wick 262 each extend through the sidewalls of the aerosol generation chamber 280 and into a respective liquid conduit 266 which is in fluid communication with the tank 252. The wick 262, saturated with aerosol precursor, may prevent gas flow passage into the liquid conduits 266 and the tank 252. Such an arrangement may allow the aerosol precursor stored in the tank 252 to convey towards the porous wick 262 through the liquid conduits 266 by gravity. The liquid conduits 266 are configured to have a hydraulic diameter that allow a controlled amount of aerosol precursor to flow from the tank 252 towards the porous wick 262. More specifically, the size of liquid conduits 266 are selected based on the rate of aerosol precursor consumption during vaporisation. For example, the liquid conduits 266 are sized to allow a sufficient amount of aerosol precursor to flow towards and replenish the wick, yet not so large as to cause excessive aerosol precursor to leak into the aerosol generation chamber. The liquid conduits 266 are configured to have a hydraulic diameter ranging from 0.01 mm to 10mm or 0.01 mm to 5mm. Preferably, the liquid conduits 266 are configured to have a hydraulic diameter in the range of 0.1mm to 1mm.

The heating filament is electrically connected to electrical contacts 256 at the base of the aerosol generation chamber 280, sealed to prevent air ingress or fluid leakage. As shown in Figure 6, when the first end of the consumable 250 is received into the main body 120, the electrical contacts 256 establish electrical communication with corresponding electrical contacts of the main body 120, and thereby allow the heater to be energised.

The vaporised aerosol precursor, or aerosol in the condensed form, may discharge from the aerosol generation chamber 280 based on pressure difference between the aerosol generation chamber 280 and the passages 270. Such pressure difference may arise form i) an increased pressure in the aerosol generation chamber 280 during vaporisation of aerosol form, and/or ii) a reduced pressure in the passage during a puff.

For example, when the heater is energised and forms a vapour, it expands in to the stagnant cavity of the aerosol generation chamber 280 and thereby causes an increase in internal pressure therein. The vaporised aerosol precursor may immediately begin to cool and may form aerosol droplets. Such increase in internal pressure causes convection inside the aerosol generation chamber which aids expulsing aerosol through the chamber outlet 282 and into the passages 270.

In the illustrated third reference arrangement, the heater is positioned within the stagnant cavity of the aerosol generation chamber 280, e.g. the heater is spaced from the chamber outlet 282. Such arrangement may reduce or prevent the amount of air flow entering the aerosol generation chamber, and therefore it may minimise the amount of turbulence in the vicinity of the heater. Furthermore, such arrangement may increase the residence time of vapour in the stagnant aerosol generation chamber 280, and thereby may result in the formation of larger aerosol droplets. In some other variations of the third reference arrangement, the heater may be positioned adjacent to the chamber outlet and therefore that the path of vapour 414 from the heater to the chamber outlet 282 is shortened. This may allow vapour to be drawn into or entrained with the air flow in a more efficient manner.

The junction inlet at each of the junctions 290 opens in a direction orthogonal or non-parallel to the air flow. That is, the junction inlet each opens at a sidewall of the respective passages 270. This allows the vapour and/or aerosol from the aerosol generation chamber 280 to entrain into the air flow at an angle, and thus improving localised mixing of the different streams, as well as encouraging aerosol formation. The aerosol may be fully formed in the air flow and be drawn out through the outlet at the mouthpiece.

With the absence of, or much reduced, air flow in the aerosol generation chamber, the aerosol as generated by the illustrated third reference arrangement has a median droplet size dso of at least 1 µm. More preferably, the aerosol as generated by the illustrated third reference arrangement has a median droplet size dso of ranged between 2µm to 3µm.

The present invention is an improvement, not visible in Figures 1-6, that can be applied to each of the described reference arrangements (and of course to many more). Each of their features can therefore be understood as, in effect, features of embodiments of the present invention.

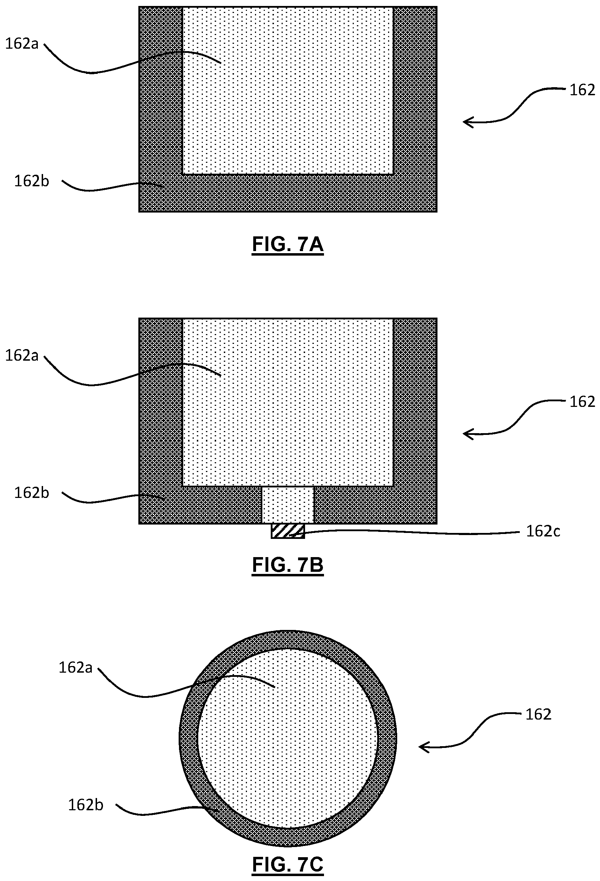

In particular, the present invention is a development of the wick 162, 262 illustrated in Figures 1-6. For ease of reference it is labelled 162 in Figures 7A-7C.

The wick of the present invention includes two materials; the first wicking material 162a being adapted to preferentially transport liquid as compared to the second wicking material 162b. In particular, the first wicking material may have a greater porosity than the second wicking material, or an average pore size more suited to liquid transport by capillary action than that of the second wicking material.

The increased porosity of the first wicking material may be achieved in a variety of ways. For example, the first material may have a greater number of pores than the second material (each pore being of similar size); and/or, the first material may have pores of a larger average diameter than those of the second material (the number of those pores being similar between the two materials).

In this regard porosity broadly means the percentage of the material volume which is void. It may be measured by standard methods, such as by mercury intrusion, as may the (mean, number) average pore diameter. See, for example, ISO 15901.

In general, as explained above and illustrated throughout the Figures, the wick 162, 262 has two significant regions: a first region which is positioned in fluid contact with the reservoir or tank containing the vaporizable e-liquid (this region providing the 'starting point' for the wicking action which transports liquid through the wick by capillary action), and a second region which is in contact or near-contact with the heater/heating element (this region providing the vaporization location, where by action of the heater liquid held by the wick is vaporized for entrainment in the air flow over the wick in the vaporization chamber).

Liquid flows (is transported) to the second region from the first region by capillary action.

In the present invention, these first and second regions are made from a first wicking material; that material is adapted for its purpose of liquid transport from the first region to the second region. The pathway from the first region to the second region, where those regions are not coterminous, is also made from the first wicking material.

The present invention provides a wick having a second wicking material, in contact with the first wicking material. The second wicking material is adapted for a different purpose; in particular to prevent liquid loss from the first wicking material for example by leakage from its pores. In the present invention it is adapted by having a lower porosity than the first wicking material; this means that liquid transport is preferentially through the first wicking material. For example, the second wicking material may preferably have an average pore diameter which is less than the average pore diameter of the first wicking material.

In some embodiments, then, the second wicking material acts to 'seal' the first wicking material against potential liquid loss. It may therefore be formed at the outer periphery of the first wicking material, effectively forming an outer casing which at least partially encloses the first wicking material.

That is, the outer surface of some or all of the first wicking material may carry the second wicking material.

It will of course be apparent that the first wicking material should remain in fluid contact with the reservoir, at the first region, rather than being separated from it by the second wicking material.

The second wicking material may, for example, be formed longitudinally co-axial with the first wicking material, in particular in parts of the first wicking material where the heating element is not present. In some embodiments, all the parts of the first wicking material other than the first region and the area in contact or near contact with the heating element may be 'coated' with the second wicking material, to minimise leakage.

The first and second wicking materials may be of any suitable porous wicking material; a suitable example is ceramic. Suitable ceramics include cordierite, Si-bonded SiC, recrystallized SiC, aluminum titanate, mullite, silicon nitride, SIALON, zirconium phosphate, zirconia, titania, alumina, calcia, and silica. The first and second wicking materials may be of the same material; or they may be of different materials. Suitably they are both ceramics, and most suitably they are of the same ceramic. Particularly suitable ceramics include silica, alumina and calcia. Mixtures of these materials may be used; for example a ceramic comprising silica, alumina and calcia may be useful. A particularly suitable material may comprise, for example, 85-90% by weight silica, 3-8% by weight alumina, 3-8% by weight calcia with the balance, if any, being other ceramic material(s) selected from the list above.

The first and second wicking materials may be formed as separate parts; that is, separate materials. Alternatively, they may be formed integrally, as portions of a single monolithic wick. That is, a single piece of material which has portions of differing porosity within it. There may be a step change of porosity between those two regions, for example if there are two or more distinct portions each with relatively uniform porosity but differing porosities between them. Of course there may also be multiple such step changes, corresponding in effect to the presence of further wicking materials beyond the first and second.

Alternatively there may be a gradual change in porosity from the 'first wicking material' region to the second wicking material' region; for example a gradual reduction in porosity.

The porosities and pore sizes of the first and second wicking materials may be chosen appropriately for their desired functions. In general the porosity of the first wicking material is greater than the porosity of the second wicking material.

The porosity of the second wicking material is preferably low, for example less than about 10%; more preferably less than about 5%; and even more preferably less than about 3%. The second wicking material may indeed be substantially non-porous (about 0%). This serves to prevent liquid flow through it; in such embodiments it can act to seal the first wicking material if appropriately positioned.

On the other hand, while the porosity of the first wicking material need only be higher than that of the second wicking material in order that liquid transport preferentially progresses through the first wicking material, its own porosity may preferably be for example more than about 40%, more than about 50%, more preferably more than about 60%, and even more preferably more than about 70%. A suitable porosity of the first wicking material is from about 50 to about 60%.

Since the second wicking material is intended to have limited function as a capillary action liquid transport medium, the pore diameter chosen in combination with its relatively low porosity is not particularly important or limited. Where capillary action is to be limited by pore diameter, rather than % pore volume, the average pore diameter of the second wicking material is suitably less than the average pore diameter of the first wicking material. For example, for the second wicking material, the average pore diameter may be less than about 50 nm may be suitable, less than about 30 nm or less than about 15 nm.

On the other hand, for the first wicking material, the average pore diameter may be above about 50 nm. For example, the average pore diameter may be above about 75 nm, above about 100 nm, or above about 150 nm; it may be much higher than this, for example above about 500 nm, above about 1000 nm (1 µm), above about 2 µ, above about 5 µm; above about 7 µm, or above about 10 µm. On the other hand, it may suitably be not greater than about 1000 µm, or not greater than about 50 µm. Such average pore diameters may encourage and increase the speed of capillary flow.

Schematic example embodiments of the wick of the present invention are illustrated in Figures 7A, 7B and 7C. In each Figure the first wicking material 162a, of higher porosity, can be seen in contact with the second wicking material 162b, of lower porosity.

Each Figure shows a cross section taken in the longitudinal dimension of the wick; that is, viewing along the length of the wick which is perpendicular to the direction in which air will flow across the wick (that flow direction being upstream to downstream; effectively bottom to top' in the Figures).

Figure 7A shows an example of a cross section through a first region of a wick; the first wicking material 162a is partially surrounded by the second wicking material 162b. Leakage of liquid held in the first wicking material 162a is limited, to the sides and downwards in Figures 7A, by the presence of the denser second wicking material 162b.

In this embodiment, the wick 162 has a broadly rectangular cross section; the first wicking material 162a is formed into a core with a broadly rectangular cross section which is surrounded on three sides with the second wicking material 162b.

Figure 7B shows a cross section taken through another embodiment, at a second region of the wick where a heater or heating element 162c is positioned on a portion of the first wicking material 162a which extends to the surface of the wick 162, that is, that is not covered by the second wicking material 162b. Positioning the heater 162c on this exposed portion of the first wicking material 162a means that the liquid held therein can be easily vaporized and hence entrained in the air flow over the wick 162. The heater can be made from any suitable material, such as FeSi.

Where, as in Figures 7A and 7B, there is an exposed part of the first wicking material 162a which does not carry the heater 162c (in those Figures, the upper surface of the wick and the first wicking material), it is preferred that the wick 162 is mounted or positioned in the smoking substitute apparatus such that that exposed part is on the downstream side of the wick 162. The heater 162c may preferably be mounted on the upstream side of the wick 162. As in Figure 7B, the first wicking material 162a may be partially exposed on that upstream side, in particular at location(s) carrying the heater 162c. Preferably the first wicking material 162a is not exposed on the upstream side except for those portions in contact or near contact with the heater 162c.

[The upstream side of the wick is the side of the wick which is closer to the air inlet; the downstream side of the wick is the side of the wick which is closer to the outlet.]

Figure 7C illustrates an embodiment where, in the first region where the cross section is taken, the first wicking material 162a is entirely enclosed with the second wicking material 162b. In this embodiment, the first wicking material 162a is formed as a cylindrical core, with the second wicking material 162b forming a further cylindrical 'coating' on its surface. That is, the outer surface of the wick 162 is substantially made from the second wicking material 162b.

This may be the case in other embodiments too; in general, the wick may be one where the outer surface is substantially made from the second wicking material except for where the first wicking material is exposed to contact (or near contact) the heater or heating element and/or to be in fluid contact with the reservoir or tank.

It will be recognised that the first and second wicking materials can be arranged as necessary for the desired liquid flow characteristics. For example, there may be a high porosity 'pathway' or channel formed from the first wicking material, the pathway being formed in or along a wick which is, mostly, of the second wicking material.

On the other hand it may be that most of the wick is of the first wicking material, with only a relatively small part having the second wicking material on its surface to prevent liquid leakage from those parts.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A vapour generating system for use in an electronic cigarette device, the system comprising:
a reservoir, configured to retain a vapourisable liquid;
a heating element;
a wick, comprising a first wicking material in contact with a second wicking material, wherein the first wicking material comprises a first region in fluid contact with the reservoir and a second region in contact or near-contact with the heating element, and wherein the wick is configured to transport vapourisable liquid from the first region of the first wicking material to the second region via capillary action;
**characterised in that** the first wicking material has a greater porosity than the second wicking material, such that liquid is transported preferentially through the first wicking material.

2. A vapour generating system according to claim 1, wherein the wick has a length along which the first wicking material and the second wicking material extend coaxially.

3. A vapour generating system according to claim 1 or claim 2, wherein the first and second wicking materials are ceramic.

4. A vapour generating system according to any one of the preceding claims, wherein the first and second wicking materials are separate materials.

5. A vapour generating system according to any one of claims 1 to 3, wherein the first and second wicking materials are integrally formed.

6. A vapour generating system according to claim 5, wherein the porosity of the wick gradually changes between the first wicking material and the second wicking material.

7. A vapour generating system according to claim 5, wherein the porosity of the wick has at least two distinct portions, one of which corresponds to the first wicking material and one of which corresponds to the second wicking material, wherein each portion has a substantially uniform porosity.

8. A vapour generating system according to any one of the preceding claims, wherein the porosity of the second wicking material is less than about 10%.

9. A vapour generating system according to any one of the preceding claims, wherein the porosity of the first wicking material is more than about 50%.

10. A vapour generating system according to any one of the preceding claims, wherein the average pore diameter of the second wicking material is less than the average pore diameter of the first wicking material.

11. A vapour generating system according to any one of the preceding claims, wherein the average pore diameter of the second wicking material is less than about 50 nm.

12. A vapour generating system according to any one of the preceding claims, wherein the average pore diameter of the first wicking material is above about 75 nm.

13. A smoking substitute apparatus having: an air inlet; an outlet; a flow passage formed between the air inlet and the outlet; a vapour generating system according to any one of the preceding claims; and a vaporisation chamber in communication with the flow passage, wherein at least part of the wick and the heater of the vapour generating system are positioned within the vaporization chamber.
